(19) Europäisches Patentamt / European Patent Office / Office européen des brevets

(11) **EP 4 214 718 B1**

(12) **EUROPEAN PATENT SPECIFICATION**

(45) Date of publication and mention
of the grant of the patent:
**21.01.2026 Bulletin 2026/04**

(21) Application number: **21805665.3**

(22) Date of filing: **14.09.2021**

(51) International Patent Classification (IPC):
**G16H 20/60** *(2018.01)*

(52) Cooperative Patent Classification (CPC):
**G16H 20/60**

(86) International application number:
**PCT/LV2021/050010**

(87) International publication number:
**WO 2022/060210 (24.03.2022 Gazette 2022/12)**

(54) **METHOD AND SYSTEM FOR A MEAL PLAN GENERATION**

VERFAHREN UND SYSTEM ZUR ERSTELLUNG EINES MAHLZEITPLANS

PROCÉDÉ ET SYSTÈME PERMETTANT LA GÉNÉRATION D'UN PLAN DE REPAS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.09.2020 LV 200062**

(43) Date of publication of application:
**26.07.2023 Bulletin 2023/30**

(73) Proprietor: **Nutrameg, SIA**
**1050 Riga (LV)**

(72) Inventor: **BALODE, Elina**
**1004 Riga (LV)**

(74) Representative: **AAA Law Latvia**
**Citadeles street 12**
**1010 Riga (LV)**

(56) References cited:
**CN-A- 111 584 039     US-A1- 2014 287 384**

**Description**

TECHNICAL FIELD

The invention relates to a method for generating a meal plan and a system implementing the method.

BACKGROUND ART

Chinese patent application publication CN110379487 discloses nutrition plan management system, which operates the nutrition plan management model which automatically supplies an accurate nutrition plan to the user according to the user data. The system does not provide the feedback system and monitoring the effectiveness of the nutrition plan.

Chinese patent application publication CN109545327 describes a diet managing method and a diet managing device. The user daily kcal amount is calculated basing only on the body mass index, without taking in account user's lifestyle. Also the feedback system is not provided. The feedback system is provided by Japanese patent application JP2016538617, which describes a diet compliance system. But this document does not disclose specific steps of the diet plan generation process.

US patent application publication US2009/0144081 describes a method and system that provides dynamic meal plan generation comprising determining meal plan servings, receiving meal plan factors and selecting recipes in accordance with the meal plan factors, and outputting the selected recipes as a meal plan. Ready to use recipes are used without adjusting the content amounts. The document only briefly describes a generation of the meal plan not stating specific steps.

US patent publication US7361143 describes a weight control software system and method having varying meal plans and meal planning schemes, but does not allow user to switch an individual recipe, only the whole meal plan is alterable. Also the modification of the recipes provided is not disclosed.

US patent application publication US2020/0074884 discloses a method of operating a meal plan optimization algorithm that can adjust and substitute meal components and then generate a meal day plan from the individual meals. The algorithm described does not allow the person skilled in the art to distinguish separate steps and does not teach how exactly the portion size is adjusted.

Other useful prior art to understand the invention can be found in US2014/287384A1 and CN111584039A.

The purpose of the present invention is to provide a method for generating a meal plan and a system operating according to the method that would provide adjustable recipes, alterable meal plans and provide a feedback.

SUMMARY OF THE INVENTION

To achieve this purpose, the disclosure adopts the following technical solutions.

There is provided a method for generating a meal plan and a system operating according to the method.

The method comprises several steps, each executed by special unit of the system.

Data are collected from a human subject in interest by the user device and activity monitoring device, where a display, a memory and a processor of the user device are connected together and the memory is configured to store data for performing the method for meal plan generation, wherein the processor is connected to the first communication unit, the display and the memory and is configured to perform the method for meal plan generation. The display, the memory and the processor communicate between each other to collect data from user, selected from a goal of the human subject, wherein the goal is selected from weight maintenance, weight loss and weight gain; a list comprising food product preferences; and daily meal diversity, wherein diversity is selected from diverse with no meal repeating more often than once in a 4 day period, medium diversity with no meal repeating more often than once in a 3 day period and low diversity with no meal repeating more often than once in a 2 day period; and a meal type, wherein the meal type is selected from breakfast, lunch, dinner and snack, and meal preferences, wherein meal preferences are selected from the meal which is preparable within 10 minutes and the meal which is preparable from 10 to 60 minutes, and an amount how many times in a week the human subject does sport activities, and age, and height, and present weight, and sex and preferred weight. Further the collected data are sent to the first communication device and through the internet communicated to a second communication unit, which is configured to communicate with the data collection unit.

A storage unit stores and provides recipes for meals, such as breakfast, lunch, dinner and snacks. Recipes are stored and provided with products. The storage unit is configured to communicate with the marking unit.

Collected from the storage unit and marked for the method by the marking unit, marked recipes with products are provided, wherein recipes are marked by one or more goal categories selected from a weight maintenance, a weight loss and a weight gain, and meal preferences selected from a meal preparable within 10 minutes and a meal preparable from 10 to 60 minutes, and meal plan preferences selected from the group of vegan meal plan, vegetarian meal plan, meal plan consisting of all foods, meal plan consisting of all foods except meat and paleo meal plan. In this step products are also marked by the amount of their nutritional facts wherein stating the amount of carbs, carbs-fibre, fibre, proteins and fats they contain in 100 g of uncooked product and then products are marked as a source of carbs, carbs-fibre, fibre, protein or fat, depending on which nutritional value they contain most. Further the kcal amount for each meal is set to predetermined kcal amount: for breakfast 35 %, for lunch 35 %, for dinner 20 % and for snack 10 %, based on the total amount of the daily kcal. Further the data from marking unit are sent to a scaling unit.

Daily kcal amount is calculated by daily kcal calculation unit, which is configured to communicate with the data collection unit and the scaling unit and calculate a daily kcal amount according to the goal communicated by the data collection unit. The daily kcal amount for a human subject, whose goal category is weight loss, is calculated using a formula (I):

$$\text{Daily kcal} = \text{BMR} \times 1,2 - \Delta, \qquad \text{(Formula I)}$$

wherein the $\Delta$ is baseline kcal difference, set depending on the goal category of the human subject.

BMR is Basal Metabolic Rate, measured in kcal, wherein BMR is calculated using data collected by the user's device by formula (II) or by formula (III), wherein formula (II) is used for a man and formula (III) is used for a woman:

$$\text{BMR (kcal)} = 66,47 + (13,75 \times \text{weight, kg}) + (5 \times \text{height, cm}) - (6,76 \times \text{age, years})$$
$$\text{(Formula II)}$$

$$\text{BMR (kcal)} = 655,1 + (9,56 \times \text{weight, kg}) + (1,85 \times \text{height, cm}) - (4,68 \times \text{age, years})$$
$$\text{(Formula III)}.$$

The daily kcal amount for a human subject, whose goal category is weight gain, is calculated using a formula (IV):

$$\text{Daily kcal} = \text{BMR} \times 1,4 + \Delta, \qquad \text{(Formula IV)}$$

wherein the $\Delta$ is baseline kcal difference, set depending on the goal category.

BMR is Basal Metabolic Rate, measured in kcal, wherein BMR is calculated using data collected by the user device by formula (II) or by formula (III), wherein formula (II) is used for a man and formula (III) is used for a woman:

$$\text{BMR (kcal)} = 66,47 + (13,75 \times \text{weight, kg}) + (5 \times \text{height, cm}) - (6,76 \times \text{age, years})$$
$$\text{(Formula II)}$$

$$\text{BMR (kcal)} = 655,1 + (9,56 \times \text{weight, kg}) + (1,85 \times \text{height, cm}) - (4,68 \times \text{age, years})$$
$$\text{(Formula III)};$$

The daily kcal amount for a human subject, whose goal category is weight maintenance is calculated using a formula (V):

$$\text{Daily kcal} = \text{BMR} \times 1,3 + \Delta, \qquad \text{(Formula V)}$$

wherein the $\Delta$ is baseline kcal difference, set depending on the goal category and wherein for the human subject, whose goal category is weight loss, at baseline the kcal difference is set to 400-500 if the human subject has not indicated any sporting activities or to 250-350 if the human subject has indicated that the human subject is doing sports 3 or more times a week or is breastfeeding.

For the human subject, whose goal category is weight gain, at baseline the kcal difference is set to 350-450 if the human subject has not indicated any sporting activities or to 500-600 if the human subject has indicated that the human subject is doing sports 3 or more times a week or is breastfeeding.

For the human subject, whose goal category is weight maintenance, at the baseline kcal difference is set to 0.

BMR is Basal Metabolic Rate, wherein BMR is calculated using data collected by user device by formula (II) or by formula (III), wherein formula (II) is used for a man and formula (III) is used for a woman:

$$BMR \text{ (kcal)} = 66{,}47 + (13{,}75 \text{ x weight, kg}) + (5 \text{ x height, cm}) - (6{,}76 \text{ x age, years})$$
(Formula II)

$$BMR \text{ (kcal)} = 655{,}1 + (9{,}56 \text{ x weight, kg}) + (1{,}85 \text{ x height, cm}) - (4{,}68 \text{ x age, years})$$
(Formula III).

A recipe is chosen from the recipes in storage unit, communicated to the marking unit, marked to conform the goal category, wherein if the goal is to maintain weight, a recipe from the weight maintenance category is selected, for the weight loss goal if the goal is to lose weight, a recipe from the weight loss category is selected, and, for the weight gain goal if the goal is to gain weight, a recipe from the weight gain category is selected.. Further the marked recipe is sent to the scaling unit.

The recipe is scaled to fit the daily kcal amount obtained by the daily kcal calculation unit and procedure is repeated for each meal thereby obtaining a meal plan. The scaling further comprises a calculation of a total amount of kcal for the marked recipe, based on the ingredient amount and their caloric value communicated by the marking unit and then a calculation of the difference between the marked recipe and needed kcal amount based on daily kcal amount communicated by the daily kcal calculation unit and on the set kcal amount for the meal in interest, and identifying ingredients of the recipe as classified into one of the following categories: carbs, carbs-fibre, fibre, proteins and fats, provided by the marking unit. The scaling can be effected into two modes:

(1) if the calculated daily kcal amount exceeds the kcal amount of the selected marked recipes, then amount of carbs is decreased by no more than 70% of its initial nutritional value, that is measured in kcal, in the recipes, if still the calculated daily kcal amount exceeds the kcal amount of the selected marked recipes, then amount of fats is decreased by no more than 70% of its initial nutritional value in the recipes, if still the calculated daily kcal amount exceeds the kcal amount of the selected marked recipes, then amount of carbs-fibre is decreased by no more than 70% of its initial nutritional value in the recipes, and if still the calculated daily kcal amount exceeds the kcal amount of the selected marked recipes, then amount of proteins is decreased by no more than 70% of its initial nutritional value in the recipes; and

(2) if the calculated daily kcal amount is below the kcal amount of the selected marked recipes, then amount of proteins is increased by no more than 300% of its initial nutritional value in the recipes, if still the calculated daily kcal amount is below the kcal amount of the selected marked recipes, then amount of carbs-fibre is increased by no more than 300% of its initial nutritional value in the recipes, if still the calculated daily kcal amount is below the kcal amount of the selected marked recipes, then amount of carbs is increased by no more than 300% of its initial nutritional value in the recipes, and if still the calculated daily kcal amount is below the kcal amount of the selected marked recipes, then amount of fats is increased by no more than 300% of its initial nutritional value in the recipes.

Products classified as fibre are excluded from the scaling process.
The obtained meal plan is communicated by the scaling unit to the second communication unit and further through internet to the first communication unit, then to the processor and then to the display.
The user can choose to switch any recipe from the meal plan communicated to him by the display. In the case the switch is necessary, the information is sent to the data collection unit and all defined steps are to be made to get a new recipe, which is scaled and sent to the second communication unit by the scaling unit.
Data provided by the user are collected by the data collection unit daily and communicated to the monitoring unit, which monitors and analyses the effectiveness of the meal plan. The result of analysing the effectiveness is classified as effective if:

a) the weight loss for a human subject, whose goal category is weight loss, is at least 1 kg per 7 days;

b) the weight gain for a human subject, whose goal category is weight gain, is at least 1 kg per 30 days;

c) the weight for a human subject, whose goal category is weight maintenance, stays at the preferred weight communicated by the data collection unit,

wherein effectiveness is classified as ineffective if:

a) the weight loss for the human subject, whose goal category indicated in step (iv) is weight loss, is less than 1 kg per 7 days;

b) the weight gain for the human subject, whose goal category indicated in step (iv) is weight gain, is less than 1 kg per 30 days;

c) the weight change of at least 1 kg for the human subject, whose goal category indicated in step (iv) is weight maintenance, is detected.

The monitoring unit communicates with the suggesting unit and a proposal for the generation of a new meal plan is communicated to a human subject in the case goal category is weight loss, when any of the following events has occurred:

a) the meal plan is classified as ineffective by the monitoring unit;

b) the amount of active energy for the last 7 days has decreased by 40 % compared to the 7 days prior to the analyzed 7 day period;

c) the amount of active energy for the last 7 days has increased by 40 % compared to the 7 days prior to the analyzed 7 day period;

d) a current weight input shows that the human subject has reached their set preferred weight; and

proposal for the generation of a new meal plan is communicated to a human subject in the case goal category is weight gain, when any of the following events has occurred:

a) the meal plan is classified as ineffective by the monitoring unit;

b) the amount of active energy for the last 7 days has increased by 40 % compared to the 7 days prior to the analyzed 7 day period;

c) the amount of active energy for the last 7 days has decreased by 40 % compared to the 7 days prior to the analyzed 7 day period;

d) a current weight input shows that the human subject has reached their set preferred weight; and

proposal for the generation of a new meal plan is communicated to a human subject in the case goal category is weight maintenance, when any of the following events has occurred:

a) a current weight input shows that the weight is for at least 1 kg less than their set preferred weight;

b) a current weight input shows that the weight is for at least 1 kg more than their set preferred weight;

c) the amount of active energy for the last 7 days has decreased by 40 % compared to the 7 days prior to the analyzed 7 day period;

d) the amount of active energy for the last 7 days has increased by 40 % compared to the 7 days prior to the analyzed 7 day period.

The events are arranged at the prioritized order where the event in the highest position from each goal is of the highest priority and the lowest position is of the lowest priority and wherein more than one of the events are detected, a proposal for the generation of a new meal plan is communicated to a human subject , basing on the event with higher priority.

If the new meal plan is to be proposed by the suggesting unit, the suggesting unit communicates with the data collection unit and the data collection unit sends the proposal for the generation of a new meal plan . The user accepts the proposal, information is communicated to the data collection unit and further to a changing unit.

The new meal plan for kcal reduction is generated when the proposal is accepted by the human subject and the current weight input shows that the weight is for at least 1 kg more than their preferred weight, wherein the generation of the new plan for kcal reduction comprises collecting the data of current weight and weight and planned physical activity level from the human subject, for whom the new meal plan for kcal reduction is generated and calculating the new meal plan according to Formula (VI):

$$\text{Daily kcal} = \text{Basal Metabolic Rate (BMR)} \times k - \Delta - 100, \qquad \text{Formula (VI)}$$

wherein:

k is users predetermined daily activity coefficient;

∆ is last calculated kcal difference;

∆ -100 is total kcal difference for user;

maximum total kcal difference can be in a range of 600 to 1000 kcal;

the new meal plan for kcal increase is generated when the proposal is accepted by the human subject and the current weight input shows that the weight is for at least 1 kg less than their preferred weight, wherein the generation of the new plan for kcal increase comprises collecting the data of current weight and weight and planned physical activity level from the human subject, for whom the new meal plan for kcal increase is generated and calculating the new meal plan according to Formula (VII):

$$\text{Daily kcal} = \text{Basal Metabolic Rate (BMR)} \times k - \Delta + 100, \qquad \text{Formula (VII)}$$

wherein:

k is users predetermined daily activity coefficient;

∆ is last calculated kcal difference;

∆ -100 is total kcal difference for user;

maximum total kcal difference can be a range of 800 to 1200 kcal;

the new meal plan for weight maintenance is generated when the proposal is accepted by the human subject and the current weight input shows that the human subject has reached their preferred weight, wherein the generation of the new plan for weight maintenance comprises collecting the data of current weight and weight and planned physical activity level from the human subject, for whom the new meal plan for weight maintenance is generated and calculating the new meal plan according to Formula (V), wherein maximum total kcal difference can be in a range of 600 to 1000 kcal, and after the generation of the new meal plan, weight and activity data collected only after the generation of the new meal plan are used for monitoring and analysis by monitoring unit.

The new meal plan is generated and communicated by the changing unit to the scaling unit to be scaled.

The changed meal plan to be scaled is received by the scaling unit and the recipes received from the marking unit are scaled to fit the daily kcal amount obtained in the daily kcal calculation unit for each meal and the changed meal plan is obtained. The new, changed and scaled meal plan is communicated to the second communication unit.

After the generation of the new meal plan weight and activity data collected only after the generation of the new meal plan are used for monitoring and analysis.

Further the signal is provided by the second communication unit to the human subject about necessity to prepare and perform breakfast, lunch, dinner or snack, and wherein the signal is adjusted depending on the selected meal preference for breakfast, lunch, dinner or snack.

BRIEF DESCRIPTION OF DRAWINGS

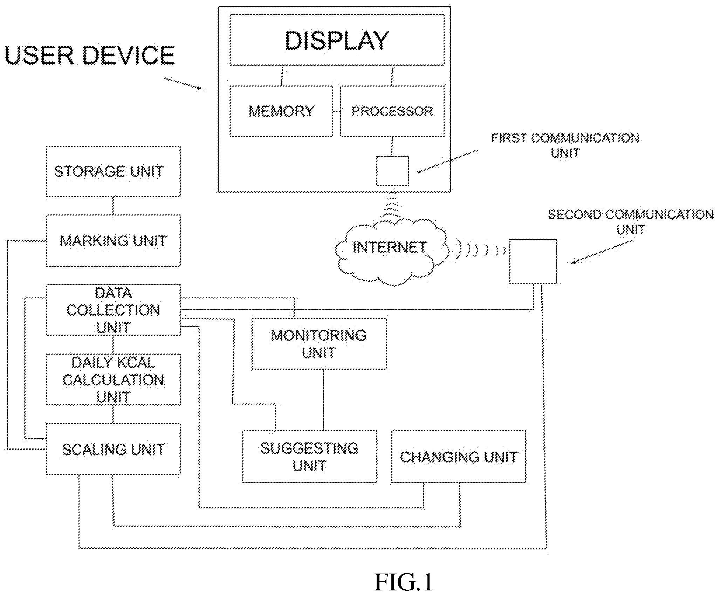

FIG. 1 is a schematic diagram illustrating system provided by the present invention.

DETAILED DESCRIPTION

The invention is described but not limited by the following examples.

The human subject in interest communicates to the user device that he wants to lose weight and does not do any sport activities, communicates that she is 57 years old female, weight is 61.1 kg, height is 150 cm and preferred weight is 58 kg. The selected meal is lunch. Further the collected data are sent to the first communication unit and through the internet communicated to a second communication unit, which is configured to communicate with the data collection unit.

The data collection unit communicates with the daily kcal calculation unit and daily kcal amount is calculated:

$$\text{BMR, kcal} = 655.1 + (9.56 \times 61.1 \text{ kg}) + (1.85 \times 150 \text{ cm}) - (4.68 \times 57 \text{ years}) = 1250 \text{ kcal}$$

$$\text{Daily kcal} = 1250 \times 1.2 - 500 = 1000 \text{ kcal}$$

The meal in interest is lunch, so the adaptation unit communicates that 35 % of the daily kcal amount has to be calculated and a result is 350 kcal.

Recipes and product nutritional values are communicated by the storage unit to the marking unit; the marking unit marks recipes and chooses a recipe from those marked as weight loss. The recipe marked: 150 g boiled potatoes, 100 g cottage cheese and 30 g yogurt. The recipe originally contains 468 kcal. The marking unit classifies products by the ingredient type:

carbs: potatoes;
protein: cottage cheese, yogurt.

The marking unit communicate these data to the scaling unit. The daily kcal calculation unit communicate with the scaling unit and the scaling unit receives information that the recipe has to be scaled to 350 kcal. A caloric value has to be reduced by 118 kcal.

The scaling unit starts with carbs. There are 116 kcal from 150 g potatoes (potatoes are the only carbs). The scaling unit reduces potatoes by 78 kcal and comes to the max limit of 70 % reduction. So, there are 118-78 kcal=40 kcal missing from the required reduction of 118 kcal. The scaling unit has to look further.

As for the second nutrition group, the scaling unit looks for fats. There are no fats in the recipe, so the scaling unit looks for the third category for reduction - products marked as carbs-fibre. There are no products marked as carbs-fibre either, so the scaling unit looks for products marked as protein. Products marked as protein are cottage cheese and yogurt. The marked recipe contains 118 kcal of products marked as protein. So, to get the required reduction for extra 40 kcal, the scaling unit needs a 34 % reduction for each of the products. 34 % < 70 %, so the scaling unit will get it done here.

The scaling unit reduces cottage cheese by 34 % from marked 100 g/98 kcal to 65 g/68 kcal and yogurt by 34 % from marked 30 g/20 kcal to 10 g/14 kcal.

The result is scaled recipe: 45 g potatoes, 65 g cottage cheese and 10 g yogurt. The scaling unit sends the recipe to the second communication unit and the recipe through the internet is sent to the first communication unit and communicated to the display to the human subject in interest, who can accept or switch the recipe. The recipe is accepted and the monitoring unit continues to monitor the meal plan effectivity, using data provided by the human subject in interest and communicated to the monitoring unit by the data collection unit.

After seven days the monitoring unit logs that user hasn't lost weight and has increased her physical activity level by more than 40 %. Each of these events is followed by a different nutrition plan: if user hasn't lost enough weight, the suggesting unit suggests to decrease the amount of kcal in the meal plan. If user has started to do sports more, the suggesting unit suggest to increase the amount of kcal in the meal plan. The suggesting unit suggests to change a meal plan. A proposal of a new meal plan is communicated to the user. The user accepts the new meal plan, the acceptance is communicated to the data collection unit that communicates with the changing unit. The changing unit checks that slow weight loss is of higher priority than increased active energy and creates a nutrition plan by decreasing the amount of kcal. The new meal plan is communicated to the scaling unit and after scaling communicated to the user.

While the invention may be susceptible to various modifications and alternative forms, specific embodiments of which have been shown by way of example in the figures and have been described in detail herein, it should be understood that the invention is not intended to be limited to the particular forms disclosed. Rather, the invention includes all modifications, equivalents, and alternatives falling within the scope of the invention as defined by the following claims.

**Claims**

1. A computer device implemented method for generating a meal plan, the method comprising the following steps:

(i) marking products by the amount of their nutritional facts wherein stating the amount of carbs, carbs-fibre, fibre, proteins and fats they contain in 100 g of uncooked product and marking the product as a source of carbs, carbs-fibre, fibre, protein or fat, depending on which nutritional value they contain most;

(ii) providing marked recipes with products, wherein recipes are marked by one or more goal categories selected from a weight maintenance, a weight loss and a weight gain, and meal preferences selected from a meal preparable within 10 minutes and a meal preparable from 10 to 60 minutes, and meal plan preferences selected from the group of vegan meal plan, vegetarian meal plan, meal plan consisting of all foods, meal plan consisting of all foods except meat and paleo meal plan;

(iii) setting the kcal amount for each of four meals within a day, wherein the kcal amount for breakfast is 35 %, for lunch is 35 %, for dinner is 20 % and for snack is 10 %, based on the total amount of the daily kcal;

(iv) collecting data from a human subject in interest, wherein the collected data is:

  a) a selected goal of the human subject, wherein the goal is selected from weight maintenance, weight loss and weight gain;

  b) a list comprising food product preferences;

  c) a preferred daily meal diversity, wherein diversity is selected from diverse with no meal repeating more often than once in a 4 day period, medium diversity with no meal repeating more often than once in a 3 day period and low diversity with no meal repeating more often than once in a 2 day period; and

  d) a meal type, wherein the meal type is selected from breakfast, lunch, dinner and snack, and

  e) meal preparation time preferences, wherein meal preparation time preferences are selected from the meal which is preparable within 10 minutes and the meal which is preparable from 10 to 60 minutes, and

  f) an amount how many times in a week the human subject does sport activities, and

  g) age, and

  h) height, and

  i) present weight, and

  j) sex and

  k) preferred weight;

(v) calculating a daily kcal amount, wherein the daily kcal amount for the human subject, whose goal indicated in step (iv) is weight loss, is calculated using a formula (I):

$$\text{Daily kcal} = \text{BMR x } 1.2 - \Delta, \qquad \text{(Formula I)}$$

wherein:

  the $\Delta$ is baseline kcal difference, set depending on the goal category of the human subject and BMR is Basal Metabolic Rate, measured in kcal, wherein BMR is calculated using data collected in step (iv) by formula (II) or by formula (III), wherein formula (II) is used for the human subject which is a man and formula (III) is used for the human subject which is a woman:

$$\text{BMR (kcal)} = 66.47 + (13.75 \text{ x weight, kg}) + (5 \text{ x height, cm}) - (6.76 \text{ x age, years})$$
$$\text{(Formula II)}$$

$$\text{BMR (kcal)} = 655.1 + (9.56 \text{ x weight, kg}) + (1.85 \text{ x height, cm}) - (4.68 \text{ x age, years})$$
$$\text{(Formula III);}$$

  the daily kcal amount for the human subject, whose goal category is weight gain, is calculated using a formula (IV):

$$\text{Daily kcal} = \text{BMR x } 1.4 + \Delta, \qquad \text{(Formula IV)}$$

  the daily kcal amount for the human subject, whose goal category is weight maintenance, is calculated using a formula (V):

$$\text{Daily kcal} = \text{BMR x } 1.3 + \Delta, \qquad \text{(Formula V)}$$

  wherein for the human subject, whose goal category is weight loss, at baseline the kcal difference is set to 400-500 if the human subject has not indicated any sporting activities in step (iv) or to 250-350 if the human subject has indicated in step (iv) that the human subject is doing sports 3 or more times a week or is breastfeeding;

  for the human subject, whose goal category is weight gain, at baseline the kcal difference is set to 350-450 if the human subject has not indicated any sporting activities in step (iv) or to 500-600 if the human subject has indicated in step (iv) that the human subject is doing sports 3 or more times a week or is breastfeeding; for the human subject, whose goal category is weight maintenance, at the baseline kcal difference is set to 0;

(vi) selecting a recipe from step (ii) according to the collected data from step (iv) regarding selected goal, wherein, if the goal is to maintain weight, a recipe from the weight maintenance category is selected, if the goal is to lose weight, a recipe from the weight loss category is selected, and, if the goal is to gain weight, a recipe from the weight gain category is selected;

(vii) scaling the marked recipe to fit the daily kcal amount obtained in step (v) for each meal and setting a meal plan, wherein the scaling further comprises a calculation of a total amount of kcal for the marked recipe provided in step (i), based on the ingredient amount and their caloric value and then a calculation of the difference between the marked recipe and needed kcal amount based on daily kcal amount obtained in step (v), and identifying ingredients of the recipe as classified into one of the following categories: carbs, carbs-fibre, fibre, proteins and fats, wherein the scaling can be effected into two following modes:

(1) if the calculated daily kcal amount exceeds the kcal amount of the selected marked recipes, then amount of carbs is decreased by no more than 70% of its initial nutritional value, that is measured in kcal, in the recipes, if still the calculated daily kcal amount exceeds the kcal amount of the selected marked recipes, then amount of fats is decreased by no more than 70% of its initial nutritional value in the recipes, if still the calculated daily kcal amount exceeds the kcal amount of the selected marked recipes, then amount of carbs-fibre is decreased by no more than 70% of its initial nutritional value in the recipes, and if still the calculated daily kcal amount exceeds the kcal amount of the selected marked recipes, then amount of proteins is decreased by no more than 70% of its initial nutritional value in the recipes; and

(2) if the calculated daily kcal amount is below the kcal amount of the selected marked recipes, then amount of proteins is increased by no more than 300% of its initial nutritional value in the recipes, if still the calculated daily kcal amount is below the kcal amount of the selected marked recipes, then amount of carbs-fibre is increased by no more than 300% of its initial nutritional value in the recipes, if still the calculated daily kcal amount is below the kcal amount of the selected marked recipes, then amount of carbs is increased by no more than 300% of its initial nutritional value in the recipes, and if still the calculated daily kcal amount is below the kcal amount of the selected marked recipes, then amount of fats is increased by no more than 300% of its initial nutritional value in the recipes;

(viii) monitoring and analyzing the effectiveness of the set meal plan, wherein the monitoring is effected by requesting the human subject to provide current weight data and amount of active energy spent every day, wherein effectiveness is classified as effective if:

a) the weight loss for the human subject, whose goal category indicated in step (iv) is weight loss, is at least 1 kg per 7 days;
b) the weight gain for the human subject, whose goal category indicated in step (iv) is weight gain, is at least 1 kg per 30 days;
c) the weight for the human subject, whose goal category indicated in step (iv) is weight maintenance, stays at the set preferred weight from step (iv),
wherein effectiveness is classified as ineffective, if:

a) the weight loss for the human subject, whose goal category indicated in step (iv) is weight loss, is less than 1 kg per 7 days;
b) the weight gain for the human subject, whose goal category indicated in step (iv) is weight gain, is less than 1 kg per 30 days;
c) the weight change of at least 1 kg for the human subject, whose goal category indicated in step (iv) is weight maintenance, is detected;

(ix) proposing a change of the meal plan of step (vii), wherein the step (ix) includes the following sub-steps:

proposal for the generation of a new meal plan is communicated to a human subject in the case goal category indicated in step (iv) is weight loss, when any of the following events has occurred:

a) the meal plan is classified as ineffective according to step (viii)
b) the amount of active energy for the last 7 days has decreased by 40 % compared to the 7 days prior to the analyzed 7 day period;
c) the amount of active energy for the last 7 days has increased by 40 % compared to the 7 days prior to the analyzed 7 day period;
d) a current weight input shows that the human subject has reached their set preferred weight; and

proposal for the generation of a new meal plan is communicated to a human subject in the case goal category indicated in step (iv) is weight gain, when any of the following events has occurred:

a) the meal plan is classified as ineffective;
b) the amount of active energy for the last 7 days has increased by 40 % compared to the 7 days prior to the analyzed 7 day period;
c) the amount of active energy for the last 7 days has decreased by 40 % compared to the 7 days prior to the analyzed 7 day period;
d) a current weight input shows that the human subject has reached their set preferred weight; and

proposal for the generation of a new meal plan is communicated to a human subject in the case goal category indicated is weight maintenance, when any of the following events has occurred:

a) a current weight input shows that the weight is for at least 1 kg less than their set preferred weight;
b) a current weight input shows that the weight is for at least 1 kg more than their set preferred weight;
c) the amount of active energy for the last 7 days has decreased by 40 % compared to the 7 days prior to the analyzed 7 day period;
d) the amount of active energy for the last 7 days has increased by 40 % compared to the 7 days prior to the analyzed 7 day period; and

wherein after the acceptance of proposal, the new meal plan is generated.

2. The method according to claim 1, wherein the method further comprises a signalizing step, in which the signal is provided to the human subject about necessity to prepare and perform breakfast, lunch, dinner or snack, and wherein the signal is adjusted depending on the selected meal preference for breakfast, lunch, dinner or snack.

3. The method according to any of the previous claims, wherein:
the new meal plan for kcal reduction is generated when the current weight input shows that the weight is for at least 1 kg more than their preferred weight and the new meal plan is accepted according to step (ix), wherein the generation of the new plan for kcal reduction comprises collecting the data of current weight and weight and planned physical activity level from the human subject, for whom the new meal plan for kcal reduction is generated and calculating the new meal plan according to Formula (VI):

$$\text{Daily kcal} = \text{Basal Metabolic Rate (BMR)} \times k - \Delta - 100, \qquad \text{Formula (VI)}$$

wherein:

k is user's predetermined daily activity coefficient;
$\Delta$ is last calculated kcal difference according to claim 1;
$\Delta$ -100 is total kcal difference for user;
maximum total kcal difference can be in a range of 600 to 1000 kcal;
the new meal plan for kcal increase is generated when the current weight input shows that the weight is for at least 1 kg less than their preferred weight, and the new meal plan is accepted according to step (ix), wherein the generation of the new plan for kcal increase comprises collecting the data of current weight and weight and planned physical activity level from the human subject, for whom the new meal plan for kcal increase is generated and calculating the new meal plan according to Formula (VII):

$$\text{Daily kcal} = \text{Basal Metabolic Rate (BMR)} \times k - \Delta + 100, \qquad \text{Formula (VII)}$$

wherein:

k is user's predetermined daily activity coefficient;

10

Δ is last calculated kcal difference according to claim 1;
Δ -100 is total kcal difference for user;
maximum total kcal difference can be a range of 800 to 1200 kcal;
the new meal plan for weight maintenance is generated when the current weight input shows that the human subject has reached their preferred weight, and the new meal plan is accepted according to step (vii), wherein the generation of the new plan for weight maintenance comprises collecting the data of current weight and weight and planned physical activity level from the human subject, for whom the new meal plan for weight maintenance is generated and calculating the new meal plan according to Formula (V), wherein maximum total kcal difference can be in a range of 600 to 1000 kcal, and after the generation of the new meal plan, weight and activity data collected only after the generation of the new meal plan are used for monitoring and analysis according to step (viii).

4. The method according to claim 1, wherein the events of Claim 1, step (ix) for each of the weight goals indicated in step (iv) are arranged at the prioritized order from a) to d), where the highest position, i.e., a), is of the highest priority and lowest position, i.e., d), is of the lowest priority and wherein more than one of the events of a) to d) are detected, the new meal plan is generated, basing on the event with higher priority.

5. A system comprising:

a device that is operated by a user, wherein the user is a human subject according to the Claim 1, wherein the user device comprises a display, a memory, a processor and a first communication unit, wherein the user device is configured to collect data from a human subject in interest, wherein the data is selected from a goal, wherein the goal is:

a) a selected goal of a human subject, wherein the goal is selected from weight maintenance, weight loss and weight gain;
b) a list comprising food product preferences; and
c) a preferred daily meal diversity, wherein diversity is selected from diverse with no meal repeating more often than once in a 4 day period, medium diversity with no meal repeating more often than once in a 3 day period and low diversity with no meal repeating more often than once in a 2 day period; and
d) a meal type, wherein the meal type is selected from breakfast, lunch, dinner and snack, and
e) meal preparation time preferences, wherein meal preparation time preferences are selected from the meal which is preparable within 10 minutes and the meal which is preparable from 10 to 60 minutes, and
f) an amount how many times in a week the human subject does sport activities, and
g) age, and
h) height, and
i) present weight, and
j) sex and
k) preferred weight,

wherein the memory is configured to store data for performing a method according to Claim 1, wherein the processor is connected to the first communication unit, the display and the memory and is configured to perform the method according to Claim 1;
wherein the first communication unit is configured to communicate data, collected by the user device through the internet to a second communication unit;
an activity monitoring device used by the human subject configured to communicate with the user device;
the second communication unit configured to communicate through the internet with the first communication unit in the user device, communicate with a data collection unit and a scaling unit, and configured to communicate data, collected by the data collection unit and the scaling unit through the internet to the first communication unit, configured to provide a signal through the internet to the first communication unit about necessity to prepare and perform breakfast, lunch, dinner or snack, and adjusting the signal depending on the selected meal preference for breakfast, lunch, dinner or snack, configured to send a proposal of changing the meal plan communicated by the data collection unit, and through the internet to the first communication unit, the data collection unit configured to communicate with the second communication unit, a monitoring unit, the scaling unit, a suggesting unit, a daily kcal calculation unit, and a changing unit, and configured to send the proposal of changing the meal plan communicated by the suggesting unit to the second communication unit, a storage unit configured to store marked recipes with products, wherein the storage unit is configured to communicate with a marking unit;
the marking unit configured to communicate with the storage unit and the scaling unit; and mark recipes by one or more goal categories selected from a weight maintenance, a weight loss and a weight gain, and meal preparation time preferences selected from a meal preparable within 10 minutes and a meal preparable from 10 to 60 minutes, and meal plan preferences selected from the group of vegan meal plan, vegetarian meal plan, meal plan consisting of all foods, meal plan consisting of all foods except meat and paleo meal plan; and mark products by the amount of their nutritional facts wherein stating the amount of carbs, carbs-fibre, fibre, proteins and fats they contain in 100 g of uncooked product and mark the product as a source of carbs, carbs-fibre, fibre, protein or fat, depending on which nutritional value they contain most; and set the kcal amount for each of four meals within a day, wherein the kcal amount for breakfast is 35 %, for lunch is 35 %, for dinner is 20 % and for snack is 10 %, based on the total amount of the daily kcal;

the daily kcal calculation unit configured to communicate with the data collection unit and the scaling unit and calculate a daily kcal amount according to the goal communicated by the data collection unit;

the scaling unit configured to communicate with the marking unit, the data collection unit, the daily kcal calculation unit, the changing unit and the second communication unit, wherein the scaling unit is configured to calculate a total amount of kcal for the marked recipe provided by the marking unit, based on the ingredient amount and their caloric value and then calculate the difference between the marked recipe and needed kcal amount based on daily kcal amount communicated by the daily kcal calculation unit, and identify ingredients of the recipe as classified into one of the following categories: carbs, carbs-fibre, fibre, proteins and fats; and communicate the meal plan to the second communication unit;

the monitoring unit configured to communicate with the data collection unit and the suggesting unit, wherein the monitoring unit is configured to monitor the data communicated by the data collection unit and analyze, whether the set meal plan is effective or ineffective and communicate the result to the suggesting unit;

the suggesting unit configured to communicate with the monitoring unit and the data collection unit, wherein the suggesting unit is configured to send a proposal of changing the meal plan to the data collection unit in the case it has received data from the monitoring unit, that the meal plan is ineffective, wherein after the proposal is accepted by the human subject in interest, the acceptance being sent through the internet to the second communication unit;

the changing unit configured to communicate with the data collection unit and the scaling unit and is configured to change the meal plan and to communicate a new meal plan to be scaled to the scaling unit, if the changing unit has communicated the acceptance of the proposal of changing the meal plan to the changing unit;

wherein the scaling unit is configured to receive the new meal plan to be scaled and scale the recipes received from the marking unit to fit the daily kcal amount obtained in the daily kcal calculation unit for each meal and obtaining the new meal plan, and communicating the new meal plan to the second communication unit.

## Patentansprüche

1. Ein computergestütztes Verfahren zur Erstellung eines Speiseplans, das die folgenden Schritte umfasst:

(i) Kennzeichnung von Produkten mit Angabe ihres Nährwerts, einschließlich des Gehalts an Kohlenhydraten, Kohlenhydratfasern, Ballaststoffen, Proteinen und Fetten, die sie in 100 g des ungekochten Produkts enthalten, sowie die Kennzeichnung des Produkts als Quelle für Kohlenhydrate, Kohlenhydratfasern, Ballaststoffe, Proteine oder Fette, je nachdem, welchen Nährwert sie am meisten enthalten;

(ii) Bereitstellung gekennzeichneter Rezepte mit Produkten, wobei die Rezepte durch eine oder mehrere Zielkategorien gekennzeichnet sind, die aus den Bereichen Gewichtsstabilisierung, Gewichtsabnahme und Gewichtszunahme ausgewählt werden können, sowie durch Essenspräferenzen, die aus Gerichten ausgewählt werden können, die innerhalb von 10 Minuten zubereitet werden können, und Gerichten, die innerhalb von 10 bis 60 Minuten zubereitet werden können, und durch Präferenzen eines Speiseplans, die aus der Gruppe der veganen Speisepläne, der vegetarischen Speisepläne, der Speisepläne, die alle Lebensmittel umfassen, der Speisepläne, die alle Lebensmittel außer Fleisch umfassen, und der Paleo-Speisepläne ausgewählt werden können;

(iii) Festlegen der Kalorienmenge für jede der vier Mahlzeiten innerhalb eines Tages, wobei die Kalorienmenge für das Frühstück 35 %, für das Mittagessen 35 %, für das Abendessen 20 % und für den Snack 10 % der täglichen Gesamtkalorienmenge beträgt;

(iv) Erheben von Daten von einer Testperson, wobei die erhobenen Daten sind:

a) das von der Testperson gewählte Ziel, wobei zwischen Gewichtserhaltung, Gewichtsabnahme und Gewichtszunahme gewählt wird;
b) eine Liste mit bevorzugten Lebensmitteln;

c) bevorzugte tägliche Ernährungsvielfalt, wobei die Vielfalt aus folgenden Optionen ausgewählt wird: abwechslungsreiche Ernährung, bei der kein Gericht häufiger als einmal innerhalb von vier Tagen wiederholt wird; mittlere Ernährungsvielfalt, bei der kein Gericht öfter als einmal innerhalb von 3 Tagen wiederholt wird; und geringe Ernährungsvielfalt, bei der kein Gericht öfter als einmal innerhalb von 2 Tagen wiederholt wird; und

d) eine Art der Mahlzeit, wobei die Art der Mahlzeit aus Frühstück, Mittagessen, Abendessen und Snack ausgewählt wird, und

e) Präferenzen hinsichtlich der Zubereitungszeit, wobei die Präferenzen hinsichtlich der Zubereitungszeit aus Mahlzeiten ausgewählt werden, die in 10 Minuten zubereitet werden können, und Mahlzeiten, die in 10 bis 60 Minuten zubereitet werden können, und

f) die Anzahl der sportlichen Aktivitäten pro Woche, die die Testperson ausübt, und

g) Alter, und

h) Körpergröße, und

i) aktuelles Gewicht, und

j) Geschlecht, und

k) bevorzugtes Gewicht;

(v) Berechnung der täglichen Kalorienmenge, wobei die tägliche Kalorienmenge für eine Testperson, deren in Schritt (iv) angegebenes Ziel die Gewichtsabnahme ist, anhand der Formel (I) berechnet wird:

$$\text{Täglicher Kalorienbedarf} = \text{BMR} \times 1{,}2 - \Delta, \text{ (Formel I)}$$

wobei:

$\Delta$ die grundlegende Kaloriendifferenz ist, die in Abhängigkeit von der Zielkategorie der Testperson festgelegt wird, und

BMR der in kcal gemessene Basalstoffwechselrate *(auf Englisch: Basal Metabolic Rate)* ist, wobei BMR unter Verwendung der in Schritt (iv) erhobenen Daten gemäß Formel (II) oder Formel (III) berechnet wird, wobei die Formel (II) für männliche Testperson und Formel (III) für weibliche Testperson verwendet wird:

$$\text{BMR (kcal)} = 66{,}47 + (13{,}75 \times \text{Gewicht, kg}) + (5 \times \text{Größe, cm}) - (6{,}76 \times \text{Alter, Jahre})$$
$$\text{(Formel II)}$$

$$\text{BMR (kcal)} = 655{,}1 + (9{,}56 \times \text{Gewicht, kg}) + (1{,}85 \times \text{Größe, cm}) - (4{,}68 \times \text{Alter, Jahre}) \text{ (Formel III)};$$

die tägliche Kalorienmenge für die Testperson, dessen Zielkategorie Gewichtszunahme ist, wird gemäß Formel (IV) berechnet:

$$\text{Täglicher Kalorienbedarf} = \text{BMR} \times 1{,}4 + \Delta, \text{(Formel IV)}$$

die tägliche Kalorienmenge für die Testperson, dessen Zielkategorie die Gewichtserhaltung ist, wird gemäß Formel (V) berechnet:

$$\text{Täglicher Kalorienbedarf} = \text{BMR} \times 1{,}3 + \Delta, \text{ (Formel V)}$$

wobei für die die Testperson, deren Zielkategorie Gewichtsabnahme ist, die Kaloriendifferenz zu Beginn auf 400-500 festgelegt wird, wenn die die Testperson in Schritt (iv) keine sportlichen Aktivitäten angegeben hat, oder auf 250-350, wenn die die Testperson in Schritt (iv) angegeben hat, dass sie 3 oder mehr Mal pro Woche Sport treibt oder stillt;

für die die Testperson, deren Zielkategorie Gewichtszunahme ist, die Kaloriendifferenz zu Beginn auf 350-450 festgelegt wird, wenn die die Testperson in Schritt (iv) keine sportlichen Aktivitäten angegeben hat, oder auf 500-600, wenn die die Testperson in Schritt (iv) angegeben hat, dass sie 3 oder mehr Mal pro Woche Sport treibt oder stillt;

für die die Testperson, deren Zielkategorie die Gewichtserhaltung ist, die Kaloriendifferenz zu Beginn auf 0 gesetzt wird;

(vi) Auswahl eines Rezepts aus Schritt (ii) gemäß den in Schritt (iv) gesammelten Daten bezüglich des ausgewählten Ziels, wobei, wenn das Ziel die Gewichtserhaltung ist, ein Rezept aus der Kategorie "Gewichtserhaltung" ausgewählt wird, wenn das Ziel die Gewichtsabnahme ist, ein Rezept aus der Kategorie "Gewichtsabnahme" ausgewählt wird, und wenn das Ziel die Gewichtszunahme ist, ein Rezept aus der Kategorie "Gewichtszunahme" ausgewählt wird;

(vii) Skalieren des markierten Rezepts, um es an die in Schritt (v) für jede Mahlzeit ermittelte tägliche Kalorienmenge anzupassen, und Festlegen eines Speiseplans, wobei das Skalieren ferner eine Berechnung der Gesamtmenge an kcal für das in Schritt (i) bereitgestellte markierte Rezept auf der Grundlage der Zutatenmenge und ihres Kalorienwerts und anschließend eine Berechnung der Differenz zwischen dem markierten Rezept und der erforderlichen Kalorienmenge auf der Grundlage der in Schritt (v) ermittelten täglichen Kalorienmenge umfasst, und Identifizieren der Zutaten des Rezepts, die in eine der folgenden Kategorien eingeteilt sind: Kohlenhydrate, Kohlenhydrate-Ballaststoffe, Ballaststoffe, Proteine und Fette, wobei die Skalierung in zwei folgenden Modi durchgeführt werden kann:

(1) wenn die berechnete tägliche Kalorienmenge die Kalorienmenge der ausgewählten markierten Rezepte überschreitet, wird die Kohlenhydratmenge um nicht mehr als 70 % ihres ursprünglichen Nährwerts, gemessen in Kalorien, in den Rezepten reduziert; wenn die berechnete tägliche Kalorienmenge immer noch die Kalorienmenge der ausgewählten markierten Rezepte überschreitet, wird die Fettmenge um nicht mehr als 70 % ihres ursprünglichen Nährwerts in den Rezepten reduziert; wenn die berechnete tägliche Kalorienmenge immer noch die Kalorienmenge der ausgewählten markierten Rezepte überschreitet, wird die Menge an Kohlenhydraten und Ballaststoffen um nicht mehr als 70 % ihres ursprünglichen Nährwerts in den Rezepten reduziert; und wenn die berechnete tägliche Kalorienmenge immer noch die Kalorienmenge der ausgewählten markierten Rezepte übersteigt, wird die Menge an Proteinen um nicht mehr als 70 % ihres ursprünglichen Nährwerts in den Rezepten verringert; und

(2) wenn die berechnete tägliche Kalorienmenge unter der Kalorienmenge der ausgewählten markierten Rezepte liegt, wird die Proteinmenge um nicht mehr als 300 % ihres ursprünglichen Nährwerts in den Rezepten erhöht; wenn die berechnete tägliche Kalorienmenge immer noch unter der Kalorienmenge der ausgewählten markierten Rezepte liegt, wird die Kohlenhydratmenge und Ballaststoffe um nicht mehr als 300 % ihres ursprünglichen Nährwerts in den Rezepten erhöht; wenn die berechnete tägliche Kalorienmenge immer noch unter der Kalorienmenge der ausgewählten markierten Rezepte liegt, wird die Menge an Kohlenhydraten um nicht mehr als 300 % ihres ursprünglichen Nährwerts in den Rezepten erhöht; und wenn die berechnete tägliche Kalorienmenge immer noch unter der Kalorienmenge der ausgewählten markierten Rezepte liegt, wird die Menge an Fetten um nicht mehr als 300 % ihres ursprünglichen Nährwerts in den Rezepten erhöht;

(viii) Überwachung und Analyse der Wirksamkeit des festgelegten Speiseplans, wobei die Überwachung dadurch erfolgt, dass die die Testperson aufgefordert wird, aktuelle Gewichtsdaten und die täglich verbrauchte aktive Energie anzugeben, wobei die Wirksamkeit als wirksam eingestuft wird, wenn:

a) die Gewichtsabnahme bei der Testperson, deren in Schritt (iv) angegebene Zielkategorie Gewichtsabnahme ist, mindestens 1 kg pro 7 Tage beträgt;
b) die Gewichtszunahme bei der Testperson, dessen in Schritt (iv) angegebene Zielkategorie Gewichtszunahme ist, mindestens 1 kg pro 30 Tage beträgt;
c) das Gewicht der Testperson, deren in Schritt (iv) angegebene Zielkategorie Gewichtsstabilisierung ist, auf dem in Schritt (iv) festgelegten Wunschgewicht bleibt, wobei die Wirksamkeit als unwirksam eingestuft wird, wenn:

a) die Gewichtsabnahme bei der Testperson, deren in Schritt (iv) angegebene Zielkategorie Gewichtsabnahme ist, weniger als 1 kg pro 7 Tage beträgt;
b) die Gewichtszunahme bei der Testperson, deren in Schritt (iv) angegebene Zielkategorie Gewichtszunahme ist, weniger als 1 kg pro 30 Tage beträgt;
c) eine Gewichtsveränderung von mindestens 1 kg bei der Testperson, deren in Schritt (iv) angegebene Zielkategorie Gewichtsstabilisierung ist, festgestellt wird;

(ix) der Vorschlag einer Änderung des Speiseplans aus Schritt (vii), wobei Schritt (ix) die folgenden Unterschritte umfasst:

Der Vorschlag für die Erstellung eines neuen Speiseplans wird der Testperson mitgeteilt, wenn das in Schritt (iv) angegebene Ziel "Gewichtsabnahme" lautet und eines der folgenden Ereignisse eingetreten ist:

a) der Speiseplan wird gemäß Schritt (viii) als unwirksam eingestuft

b) die aktive Energie der letzten 7 Tage ist im Vergleich zu den 7 Tagen vor dem analysierten 7-Tage-Zeitraum um 40 % zurückgegangen;

c) die aktive Energie der letzten 7 Tage ist im Vergleich zu den 7 Tagen vor dem analysierten 7-Tage-Zeitraum um 40 % gestiegen;

d) eine aktuelle Gewichtseingabe zeigt, dass die Testperson ihr eingestelltes Wunschgewicht erreicht hat; und ein Vorschlag für die Erstellung eines neuen Speiseplans wird der Testperson mitgeteilt, wenn das in Schritt (iv) angegebene Ziel "Gewichtszunahme" lautet und eines der folgenden Ereignisse eingetreten ist:

a) der Speiseplan wird als unwirksam eingestuft;

b) die aktive Energie der letzten 7 Tage ist im Vergleich zu den 7 Tagen vor dem analysierten 7-Tage-Zeitraum um 40 % gestiegen;

c) die aktive Energie der letzten 7 Tage ist im Vergleich zu den 7 Tagen vor dem analysierten 7-Tage-Zeitraum um 40 % zurückgegangen;

d) eine aktuelle Gewichtseingabe zeigt, dass die Testperson ihr eingestelltes Wunschgewicht erreicht hat; und ein Vorschlag, einen neuen Speiseplan zu erstellen, wird der Testperson mitgeteilt, wenn die Zielkategorie als Gewichtsstabilisierung angegeben ist und eines der folgenden Ereignisse eingetreten ist:

a) eine aktuelle Gewichtsangabe zeigt, dass das Gewicht mindestens 1 kg unter dem eingestellten Wunschgewicht liegt;

b) eine aktuelle Gewichtseingabe zeigt, dass das Gewicht mindestens 1 kg über dem eingestellten Wunschgewicht liegt;

c) die aktive Energie der letzten 7 Tage ist im Vergleich zu den 7 Tagen vor dem analysierten 7-Tage-Zeitraum um 40 % zurückgegangen;

d) die aktive Energie der letzten 7 Tage im Vergleich zu den 7 Tagen vor dem analysierten 7-Tage-Zeitraum um 40 % gestiegen ist; und

wobei nach der Annahme des Vorschlags der neue Speiseplan erstellt wird.

**2.** Verfahren nach Formel 1, wobei das Verfahren zusätzlich einen signalisierenden Schritt umfasst, bei dem der Testperson ein Signal gegeben wird, dass sie Frühstück, Mittagessen, Abendessen oder einen Snack zubereiten und zu sich nehmen soll, wobei das Signal in Abhängigkeit von den ausgewählten Präferenzen für das Frühstück, Mittagessen, Abendessen oder den Snack angepasst wird.

**3.** Verfahren nach einer der vorstehenden Formeln, wobei:
der neue Speiseplan zur Kalorienreduktion erstellt wird, wenn die aktuelle Gewichtseingabe zeigt, dass das Gewicht mindestens 1 kg über dem Wunschgewicht liegt, und der neue Speiseplan gemäß Schritt (ix) akzeptiert wird, wobei die Erstellung des neuen Plans zur Kalorienreduktion das Erheben der Daten zum aktuellen Gewicht und zum geplanten körperlichen Aktivitätsniveau von der Testperson umfasst, für die der neue Speiseplan zur Kalorienreduktion erstellt wird, sowie die Berechnung des neuen Speiseplans gemäß Formel (VI) berechnet wird:

$$\text{tägliche Kalorienmenge} = \text{Basalstoffwechselrate (BMR)} \times k - \Delta - 100, \text{Formel (VI)},$$

wobei:

$k$ der vom Benutzer vorab festgelegte tägliche Aktivitätskoeffizient ist;

$\Delta$ die zuletzt berechnete Kaloriendifferenz gemäß Formel 1 ist;

$\Delta$ -100 die Gesamtkaloriendifferenz für den Benutzer ist;

der maximale totale Kaloriendifferenz im Bereich von 600 bis 1000 kcal liegen kann;

der neue Speiseplan zur Kalorienerhöhung wird erstellt, wenn die aktuelle Gewichtseingabe zeigt, dass das Gewicht mindestens 1 kg unter dem Wunschgewicht liegt, und

der neue Speiseplan gemäß Schritt (ix) akzeptiert wird, wobei die Erstellung des neuen Plans zur Kalorienerhöhung das Erheben der Daten zum aktuellen Gewicht und zum geplanten körperlichen Aktivitätsniveau von der Testperson umfasst, für die der neue Speiseplan zur Kalorienerhöhung erstellt wird, sowie die Berechnung des neuen Speiseplans gemäß Formel (VII) berechnet wird:

$$\text{tägliche Kalorienmenge} = \text{Basalstoffwechselrate (BMR)} \times k - \Delta + 100, \text{ Formel (VII)}$$

wobei:

k der vom Benutzer vorab festgelegte tägliche Aktivitätskoeffizient ist;
$\Delta$ die zuletzt berechnete Kaloriendifferenz gemäß Formel 1 ist;
$\Delta$ -100 die Gesamtkaloriendifferenz für den Benutzer ist;
der maximale totale Kaloriendifferenz im Bereich von 800 bis 1200 kcal liegen kann;
der neue Speiseplan zur Gewichtserhaltung wird erstellt, wenn die Eingabe des aktuellen Gewichts zeigt, dass die Testperson ihr Wunschgewicht erreicht hat, und der neue Speiseplan gemäß Schritt (vii) akzeptiert wird, wobei die Erstellung des neuen Plans zur Gewichtserhaltung das Erheben der Daten zum aktuellen Gewicht und zur geplanten körperlichen Aktivität der Testperson, für die der neue Speiseplan zur Gewichtserhaltung erstellt wird, und die Berechnung des neuen Speiseplans gemäß der Formel (V) berechnet wird, wobei die maximale totale Kaloriendifferenz in einem Bereich von 600 bis 1000 kcal liegen kann, und nach der Erstellung des neuen Speiseplans die erst nach der Erstellung des neuen Speiseplans erfassten Gewichts- und Aktivitätsdaten zur Überwachung und Analyse gemäß Schritt (viii) verwendet werden.

4. Das Verfahren gemäß Formel 1, wobei die Ereignisse von Formel 1, Schritt (ix) für jedes der in Schritt (iv) angegebenen Gewichtsziele in der priorisierten Reihenfolge von a) bis d) angeordnet sind, wobei die höchste Position, d. h. a), die höchste Priorität und die niedrigste Position, d. h. d), die niedrigste Priorität hat, und wobei mehr als eines der Ereignisse von a) bis d) erkannt wird, der neue Speiseplan auf der Grundlage des Ereignisses mit höherer Priorität erstellt wird.

5. Ein System, das Folgendes umfasst:
ein Gerät, das von einem Benutzer bedient wird, wobei der Benutzer eine Testperson gemäß Formel 1 ist, wobei das Benutzergerät eine Anzeige, einen Speicher, einen Prozessor und eine erste Kommunikationseinheit umfasst, wobei das Benutzergerät so konfiguriert ist, dass es Daten von der Testperson sammelt, wobei die Daten aus einem Ziel ausgewählt werden, wobei das Ziel Folgendes ist:

a) das von der Testperson gewählte Ziel, wobei zwischen Gewichtserhaltung, Gewichtsabnahme und Gewichtszunahme gewählt wird;
b) eine Liste mit bevorzugten Lebensmitteln; und
c) bevorzugte tägliche Ernährungsvielfalt, wobei die Vielfalt aus folgenden Optionen ausgewählt wird: abwechslungsreiche Ernährung, bei der kein Gericht häufiger als einmal innerhalb von 4 Tagen wiederholt wird; mittlere Ernährungsvielfalt, bei der kein Gericht öfter als einmal innerhalb von 3 Tagen wiederholt wird; und geringe Ernährungsvielfalt, bei der kein Gericht öfter als einmal innerhalb von 2 Tagen wiederholt wird; und
d) eine Art der Mahlzeit, wobei die Art der Mahlzeit aus Frühstück, Mittagessen, Abendessen und Snack ausgewählt wird, und
e) Präferenzen hinsichtlich der Zubereitungszeit, wobei die Präferenzen hinsichtlich der Zubereitungszeit aus Mahlzeiten ausgewählt werden, die in 10 Minuten zubereitet werden können, und Mahlzeiten, die in 10 bis 60 Minuten zubereitet werden können, und
f) die Häufigkeit, mit der die Testperson pro Woche Sport treibt, und g) Alter, und
h) Körpergröße, und
i) aktuelles Gewicht, und
j) Geschlecht, und
k) bevorzugtes Gewicht,
wobei der Speicher so konfiguriert ist, dass er Daten zum Ausführen eines Verfahrens gemäß Formel 1 speichert, wobei der Prozessor mit der ersten Kommunikationseinheit, der Anzeige und dem Speicher verbunden ist und so konfiguriert ist, dass er das Verfahren gemäß Formel 1 ausführt;
wobei die erste Kommunikationseinheit so konfiguriert ist, dass sie Daten, die vom Benutzergerät erfasst wurden, über das Internet an eine zweite Kommunikationseinheit übermittelt;
ein von der Testperson verwendetes Aktivitätsüberwachungsgerät, das zur Kommunikation mit dem Benutzergerät konfiguriert ist;

die zweite Kommunikationseinheit so konfiguriert ist, dass sie über das Internet mit der ersten Kommunikationseinheit im Benutzergerät kommuniziert, mit einer Datenerfassungseinheit und einer Skalierungseinheit kommuniziert und die von der Datenerfassungseinheit und der Skalierungseinheit erfassten Daten über das Internet an die erste Kommunikationseinheit übermittelt; sie ist so konfiguriert, dass sie über das Internet ein Signal an die erste Kommunikationseinheit sendet, das die Notwendigkeit der Zubereitung und des Verzehrs von Frühstück, Mittagessen, Abendessen oder Snacks signalisiert und das Signal je nach gewählter Mahlzeitpräferenz für Frühstück, Mittagessen, Abendessen oder Snack anpasst; sie ist so konfiguriert, dass sie einen von der Datenerfassungseinheit übermittelten Vorschlag zur Änderung des Speiseplans über das Internet an die erste Kommunikationseinheit sendet; die Datenerfassungseinheit ist so konfiguriert, dass sie mit der zweiten Kommunikationseinheit, einer Überwachungseinheit, der Skalierungseinheit, einer Vorschlagseinheit, einer Einheit zur Berechnung des täglichen Kalorienverbrauchs und einer Änderungseinheit kommuniziert und den von der Vorschlagseinheit übermittelten Vorschlag zur Änderung des Speiseplans an die zweite Kommunikationseinheit sendet; eine Speichereinheit ist so konfiguriert, dass sie markierte Rezepte mit Produkten speichert, wobei die Speichereinheit so konfiguriert ist, dass sie mit einer Markierungseinheit kommuniziert;

die Markierungseinheit, die so konfiguriert ist, dass sie mit der Speichereinheit und der Skalierungseinheit kommuniziert; und Rezepte anhand einer oder mehrerer Zielkategorien, die aus Gewichtsstabilisierung, Gewichtsabnahme und Gewichtszunahme ausgewählt werden, sowie anhand von Präferenzen für die Zubereitungszeit, die aus einer innerhalb von 10 Minuten zubereitbaren Mahlzeit und einer innerhalb von 10 bis 60 Minuten zubereitbaren Mahlzeit ausgewählt werden, und anhand von Präferenzen für den Speiseplan, die aus der Gruppe veganer Speiseplan, vegetarischer Speiseplan, Speiseplan bestehend aus allen Lebensmitteln, Speiseplan bestehend aus allen Lebensmitteln außer Fleisch und Paleo-Speiseplan ausgewählt werden, kennzeichnet; und Produkte anhand ihrer Nährwertangaben kennzeichnet, wobei die Menge an Kohlenhydraten, Kohlenhydratfasern, Ballaststoffen, Proteinen und Fetten angegeben wird, die sie in 100 g des ungekochten Produkts enthalten, und das Produkt als Quelle für Kohlenhydrate, Kohlenhydratfasern, Ballaststoffe, Proteine oder Fette kennzeichnet, je nachdem, welchen Nährwert sie am meisten enthalten; und die Kalorienmenge für jede der vier Mahlzeiten innerhalb eines Tages festlegt, wobei die Kalorienmenge für das Frühstück 35 %, für das Mittagessen 35 %, für das Abendessen 20 % und für den Snack 10 % beträgt, basierend auf der Gesamtmenge der täglichen Kalorien; die Einheit zur Berechnung der täglichen Kalorienmenge so konfiguriert ist, dass sie mit der Datenerfassungseinheit und der Skalierungseinheit kommuniziert und eine tägliche Kalorienmenge gemäß dem von der Datenerfassungseinheit übermittelten Ziel berechnet;

die Skalierungseinheit so konfiguriert ist, dass sie mit der Markierungseinheit, der Datenerfassungseinheit, der Einheit zur Berechnung der täglichen Kalorienmenge, der Änderungseinheit und der zweiten Kommunikationseinheit kommuniziert, wobei die Skalierungseinheit so konfiguriert ist, dass sie eine gesamte Kalorienmenge für das von der Markierungseinheit bereitgestellte markierte Rezept auf der Grundlage der Zutatenmenge und ihres Kalorienwerts berechnet und dann dann die Differenz zwischen dem markierten Rezept und der benötigten Kalorienmenge auf der Grundlage der täglichen Kalorienmenge berechnet, die von der Einheit zur Berechnung der täglichen Kalorienmenge mitgeteilt wird, und die Zutaten des Rezepts als in eine der folgenden Kategorien eingestuft identifiziert: Kohlenhydrate, Kohlenhydrate - Ballaststoffe, Ballaststoffe, Proteine und Fette; und den Speiseplan an die zweite Kommunikationseinheit übermittelt;

die Überwachungseinheit so konfiguriert ist, dass sie mit der Datenerfassungseinheit und der Vorschlagseinheit kommuniziert, wobei die Überwachungseinheit so konfiguriert ist, dass sie die von der Datenerfassungseinheit übermittelten Daten überwacht und analysiert, ob der festgelegte Speiseplan wirksam oder unwirksam ist, und das Ergebnis an die Vorschlagseinheit übermittelt; die Vorschlagseinheit so konfiguriert ist, dass sie mit der Überwachungseinheit und der Datenerfassungseinheit kommuniziert, wobei die Vorschlagseinheit so konfiguriert ist, dass sie einen Vorschlag zur Änderung des Speiseplans an die Datenerfassungseinheit sendet, wenn sie von der Überwachungseinheit Daten erhalten hat, dass der Speiseplan unwirksam ist, wobei nach Annahme des Vorschlags durch die Testperson die Annahme über das Internet an die zweite Kommunikationseinheit gesendet wird; die Änderungseinheit, die so konfiguriert ist, dass sie mit der Datenerfassungseinheit und der Skalierungseinheit kommuniziert, und die so konfiguriert ist, dass sie den Speiseplan ändert und einen neuen Speiseplan, der skaliert werden soll, an die Skalierungseinheit übermittelt, wenn die Änderungseinheit die Annahme des Vorschlags zur Änderung des Speiseplans an die Änderungseinheit übermittelt hat; wobei die Skalierungseinheit so konfiguriert ist, dass sie den neuen zu skalierenden Speiseplan empfängt und die von der Markierungseinheit empfangenen Rezepte so skaliert, dass sie zu der in der Berechnungseinheit zur Berechnung von täglichen Kalorienmenge für jede Mahlzeit

die ermittelten täglichen Kalorienmenge anpasst, und den neuen Speiseplan erhält und diesen an die zweite Kommunikationseinheit übermittelt.

**Revendications**

1.  Un procédé mis en œuvre par un dispositif informatique pour générer un plan de repas, le procédé comprenant les étapes suivantes:

    (i) marquer les produits en fonction de leur valeur nutritive, en indiquant la quantité de glucides, glucides-fibres, fibres, protéines et de matières grasses qu'ils contiennent dans 100 g de produit non cuit et en marquant le produit comme source de glucides, glucides-fibres, fibres, protéines ou de matières grasses, selon la valeur nutritive qu'il contient le plus;
    (ii) offrir des recettes marquées avec les produits, les recettes étant marquées par une ou plusieurs catégories d'objectifs sélectionnées parmi le maintien du poids, la perte de poids et la prise de poids, et des préférences de repas sélectionnées parmi un repas qui peut être préparé en 10 minutes et un repas qui peut être préparé en 10 à 60 minutes, et des préférences de plan de repas sélectionnées parmi un groupe de plan de repas végétalien, plan de repas végétarien, plan de repas composé de tous les aliments, plan de repas composé de tous les aliments sauf la viande et plan de repas paléo;
    (iii) fixer la quantité de kcal pour chacun des quatre repas d'une journée, la quantité de kcal pour le petit-déjeuner étant de 35 %, pour le déjeuner de 35 %, pour le dîner de 20 % et pour la collation de 10 %, sur la base de la quantité totale de kcal quotidienne;
    (iv) la collecte de données auprès d'un sujet humain intéressé, les données collectées étant:

    a) un objectif choisi par le sujet humain, cet objectif étant choisi parmi le maintien du poids, la perte de poids et la prise de poids;
    b) une liste comprenant les préférences en matière des produits alimentaires;
    c) une diversité alimentaire quotidienne privilégiée, cette diversité étant choisie parmi une diversité élevée (aucun repas ne se répétant plus d'une fois sur une période de 4 jours), une diversité moyenne (aucun repas ne se répétant plus d'une fois sur une période de 3 jours) et une faible diversité (aucun repas ne se répétant plus d'une fois sur une période de 2 jours); et
    d) un type de repas, ce type de repas étant choisi parmi le petit-déjeuner, le déjeuner, le dîner et la collation, et
    e) les préférences de temps de préparation de repas, les préférences de temps de préparation de repas étant choisies parmi le repas qui peut être préparé entre 10 minutes et le repas qui peut être préparé entre 10 à 60 minutes, et
    f) le nombre de fois par semaine où le sujet humain pratique une activité sportive, et
    g) l'âge, et
    h) la taille, et
    i) le poids actuel, et
    j) le sexe et
    k) le poids préféré;

    (v) calcul d'un apport quotidien en kcal, l'apport quotidien en kcal pour le sujet humain, dont l'objectif indiqué à l'étape (iv) est la perte de poids, est calculé à l'aide d'une formule (I):

$$\text{Apport quotidien en kcal} = \text{BMR} \times 1.2 - \Delta, \text{(Formule I)}$$

    où:

    $\Delta$ représente la différence de kcal de base, définie en fonction de la catégorie cible du sujet humain et le BMR est le taux métabolique basal, mesuré en kcal, où le BMR est calculé à l'aide des données collectées à l'étape (iv) par la formule (II) ou par la formule (III), où la formule (II) est utilisée pour le sujet humain qui est un homme et la formule (III) est utilisée pour le sujet humain qui est une femme:

$$\text{BMR (kcal)} = 66,47 + (13,75 \times \text{poids, kg}) + (5 \times \text{taille, cm}) - (6,76 \times \text{âge, ans}), \text{(Formule II)}$$

BMR (kcal) = 655,1 + (9,56 x poids, kg) + (1,85 x taille, cm) - (4,68 x âge, ans), (Formule III);

l'apport quotidien en kcal pour le sujet humain, dont l'objectif est la prise de poids, est calculé à l'aide d'une formule (IV) :

Apport quotidien en kcal = BMR x 1.4 + Δ, (Formule IV)

l'apport quotidien en kcal pour le sujet humain, dont l'objectif est le maintien du poids, est calculé à l'aide d'une formule (V) :

Apport quotidien en kcal = BMR x 1.3 + Δ, (Formule V)

dans lequel, pour le sujet humain dont la catégorie cible est la perte de poids, la différence de kcal est fixée à 400-500 si le sujet humain n'a indiqué aucune activité sportive à l'étape (iv) ou à 250-350 si le sujet humain a indiqué à l'étape (iv) qu'il pratique un sport 3 fois ou plus par semaine ou qu'il allaite ;
pour le sujet humain, dont la catégorie cible est la prise de poids, la différence de kcal est fixée à 350-450 si le sujet humain n'a indiqué aucune activité sportive à l'étape (iv) ou à 500-600 si le sujet humain a indiqué à l'étape (iv) qu'il pratique un sport 3 fois ou plus par semaine ou qu'il allaite ;
pour le sujet humain, dont l'objectif est le maintien du poids, la différence de kcal de base est fixée à 0 ;

(vi) sélectionner une recette de l'étape (ii) en fonction des données collectées à l'étape (iv) concernant l'objectif sélectionné, dans lequel, si l'objectif est de maintenir le poids, une recette de la catégorie maintien du poids est sélectionnée, si l'objectif est de perdre du poids, une recette de la catégorie perte de poids est sélectionnée et, si l'objectif est de prendre du poids, une recette de la catégorie prise de poids est sélectionnée ;
(vii) adapter la recette indiquée à l'apport quotidien en kcal obtenu à l'étape (v) pour chaque repas et établir un plan de repas. Cette adaptation comprend le calcul de l'apport calorique total de la recette indiquée à l'étape (i), en fonction de la quantité d'ingrédients et de leur valeur calorique, puis le calcul de la différence entre l'apport calorique de la recette indiquée et l'apport calorique nécessaire, en fonction de l'apport calorique journalier obtenu à l'étape (v). Les ingrédients de la recette sont ensuite classés dans l'une des catégories suivantes : glucides, glucides-fibres, fibres, protéines et lipides. Cette adaptation peut être effectuée selon deux modes :

(1) Si l'apport quotidien en kcal calculé dépasse l'apport calorique des recettes sélectionnées et signalées, la quantité de glucides est réduite de 70 % maximum de leur valeur nutritionnelle initiale (exprimée en kcal) dans les recettes. Si l'apport quotidien en kcal calculé dépasse toujours l'apport quotidien en kcal des recettes sélectionnées et signalées, la quantité de lipides est réduite de 70 % maximum de leur valeur nutritionnelle initiale dans les recettes. Si l'apport quotidien en kcal calculé dépasse toujours l'apport quotidien en kcal des recettes sélectionnées et signalées, la quantité de glucides et de fibres est réduite de 70 % maximum de leur valeur nutritionnelle initiale dans les recettes. Enfin, si l'apport quotidien en kcal calculé dépasse toujours l'apport quotidien en kcal des recettes sélectionnées et signalées, la quantité de protéines est réduite de 70 % maximum de leur valeur nutritionnelle initiale dans les recettes.
(2) si l'apport quotidien en kcal calculé est inférieur à l'apport quotidien en kcal des recettes sélectionnées et marquées, alors la quantité de protéines est augmentée d'au plus 300 % de leur valeur nutritionnelle initiale dans les recettes ; si l'apport quotidien en kcal calculé est toujours inférieur à l'apport calorique des recettes sélectionnées et marquées, alors la quantité de glucides-fibres est augmentée d'au plus 300 % de leur valeur nutritionnelle initiale dans les recettes ; si l'apport quotidien en kcal calculé est toujours inférieur à l'apport calorique des recettes sélectionnées et marquées, alors la quantité de glucides est augmentée d'au plus 300 % de leur valeur nutritionnelle initiale dans les recettes ; et si l'apport quotidien en kcal calculé est toujours inférieur à l'apport calorique des recettes sélectionnées et marquées, alors la quantité de lipides est augmentée d'au plus 300 % de leur valeur nutritionnelle initiale dans les recettes.

(viii) le suivi et l'analyse de l'efficacité du plan alimentaire établi, ce suivi étant effectué en demandant au sujet humain de fournir des données actuelles sur son poids et sa dépense énergétique active quotidienne, l'efficacité étant considérée comme effective si :

a) la perte de poids du sujet humain, dont la catégorie cible indiquée à l'étape (iv) est la perte de poids, est d'au moins 1 kg par 7 jours ;
b) la prise de poids du sujet humain, dont la catégorie cible indiquée à l'étape (iv) est la prise de poids, est d'au moins 1 kg par 30 jours;

c) le poids du sujet humain, dont la catégorie d'objectif indiquée à l'étape (iv) est le maintien du poids, reste au poids préféré fixé à l'étape (iv),

où l'efficacité est considérée comme inefficace si:

a) la perte de poids du sujet humain, dont la catégorie cible indiquée à l'étape (iv) est la perte de poids, est inférieure à 1 kg par 7 jours;

b) la prise de poids du sujet humain, dont la catégorie cible indiquée à l'étape (iv) est la prise de poids, est inférieure à 1 kg par 30 jours;

c) une variation de poids d'au moins 1 kg est relevée chez le sujet humain dont la catégorie cible indiquée à l'étape (iv) est le maintien du poids;

(ix) proposant une modification du plan de repas de l'étape (vii), dans laquelle l'étape (ix) comprend les sous-étapes suivantes:

La proposition de création d'un nouveau plan alimentaire est communiquée à un sujet humain dans la catégorie d'objectif indiquée à l'étape (iv) qui est la perte de poids, lorsque l'un des événements suivants s'est produit:

a) le plan de repas est classé comme inefficace selon l'étape (viii)

b) la quantité d'énergie active des 7 derniers jours a diminué de 40 % par rapport aux 7 jours précédant la période de 7 jours analysée;

c) la quantité d'énergie active des 7 derniers jours a augmenté de 40 % par rapport aux 7 jours précédant la période de 7 jours analysée;

d) la mensuration du poids actuel montre que le sujet humain a atteint son poids préféré défini; et

la proposition de génération d'un nouveau plan de repas est communiquée à un sujet humain dans la catégorie d'objectif indiquée à l'étape (iv) qui est la prise de poids, lorsque l'un des événements suivants s'est produit:

a) le plan de repas est jugé inefficace;

b) la quantité d'énergie active au cours des 7 derniers jours a augmenté de 40 % par rapport aux 7 jours précédant la période de 7 jours analysée;

c) la quantité d'énergie active des 7 derniers jours a diminué de 40 % par rapport aux 7 jours précédant la période de 7 jours analysée;

d) la mensuration du poids actuel montre que le sujet humain a atteint son poids préféré défini; et

la proposition d'élaboration d'un nouveau plan de repas est communiquée à un sujet humain dont l'objectif est le maintien du poids, lorsque l'un des événements suivants s'est produit:

a) la mensuration de poids actuelle montre que le poids est inférieur d'au moins 1 kg à son poids préféré défini;

b) la mensuration de poids actuelle montre que le poids est supérieur d'au moins 1 kg à son poids préféré défini;

c) la quantité d'énergie active des 7 derniers jours a diminué de 40 % par rapport aux 7 jours précédant la période de 7 jours analysée;

d) la quantité d'énergie active au cours des 7 derniers jours a augmenté de 40 % par rapport aux 7 jours précédant la période de 7 jours analysée; et

après l'acceptation de la proposition, le nouveau plan de repas est généré.

2. Le procédé selon la revendication 1, dans lequel le procédé comprend en outre une étape de signalisation, dans laquelle une signalisation est donnée au sujet humain concernant la nécessité de préparer et de prendre le petit-déjeuner, le déjeuner, le dîner ou une collation, et dans lequel la signalisation est ajustée en fonction de la préférence de repas sélectionnée pour le petit-déjeuner, le déjeuner, le dîner ou une collation.

3. Le procédé selon l'une quelconque des revendications précédentes, où:

le nouveau plan de repas pour la réduction des kcal est généré lorsque le poids actuel mesuré montre que le poids est supérieur d'au moins 1 kg au poids préféré et que le nouveau plan de repas est accepté conformément à l'étape (ix), dans laquelle la génération du nouveau plan pour la réduction des kcal comprend la collecte des données du poids actuel et du niveau d'activité physique prévu du sujet humain pour lequel le nouveau plan de repas pour la réduction des kcal est généré et le calcul du nouveau plan de repas selon la formule (VI):

$$\text{Apport quotidien en kcal} = \text{taux métabolique basal (BMR)} \times k - \Delta - 100, \text{ Formule (VI)}$$

où:

k est le coefficient d'activité quotidienne prédéterminé de l'utilisateur;
$\Delta$ est la dernière différence de kcal calculée selon la revendication 1;
$\Delta$-100 est la différence kcal totale pour l'utilisateur;
la différence maximale totale de kcal peut être comprise entre 600 et 1000 kcal;
le nouveau plan de repas pour l'augmentation des kcal est généré lorsque le poids actuel mesuré indique que le poids est inférieur d'au moins 1 kg au poids souhaité, et le nouveau plan de repas est accepté conformément à l'étape (ix), dans laquelle la génération du nouveau plan pour l'augmentation des kcal comprend la collecte des données du poids actuel et du niveau d'activité physique prévu du sujet humain pour lequel le nouveau plan de repas pour l'augmentation des kcal est généré et le calcul du nouveau plan de repas selon la formule (VII):

$$\text{Apport quotidien en kcal} = \text{taux métabolique basal (BMR)} \times k - \Delta + 100, \text{ Formule (VII)}$$

où:

k est le coefficient d'activité quotidienne prédéterminé de l'utilisateur;
$\Delta$ est la dernière différence calculée de kcal selon la revendication 1;
$\Delta$-100 est la différence kcal totale pour l'utilisateur;
la différence totale maximale en kcal peut se situer entre 800 et 1200 kcal;
le nouveau plan de repas pour le maintien du poids est généré lorsque le poids actuel indique que le sujet humain a atteint son poids préféré, et le nouveau plan de repas est accepté selon l'étape (vii), dans laquelle la génération du nouveau plan pour le maintien du poids comprend la collecte des données de poids actuel et de niveau d'activité physique prévu du sujet humain pour lequel le nouveau plan de repas pour le maintien du poids est généré et le calcul du nouveau plan de repas selon la formule (V), dans laquelle la différence totale maximale de kcal peut être comprise entre 600 et 1000 kcal, et après la génération du nouveau plan de repas, les données de poids et d'activité collectées uniquement après la génération du nouveau plan de repas sont utilisées pour le suivi et l'analyse selon l'étape (viii).

4. Le procédé selon la revendication 1, dans lequel les événements de l'étape (ix) de la revendication 1 pour chacun des objectifs de poids indiqués à l'étape (iv) sont classés par ordre de priorité de a) à d), où la position la plus élevée, c'est-à-dire a), est de priorité maximale et la position la plus basse, c'est-à-dire d), est de priorité la plus basse et dans lequel plus d'un des événements de a) à d) sont relevés, le nouveau plan de repas est généré, en se basant sur l'événement ayant la priorité la plus élevée.

5. Un système comprenant:
un dispositif utilisé par un utilisateur, cet utilisateur étant un sujet humain au sens de la revendication 1, ledit dispositif comprenant un écran, une mémoire, un processeur et une première unité de communication, ledit dispositif étant configuré pour collecter des données auprès d'un sujet humain d'intérêt, lesdites données étant sélectionnées en fonction d'un objectif, ledit objectif étant:

a) un objectif choisi par un sujet humain, cet objectif étant choisi parmi le maintien du poids, la perte de poids et la prise de poids;
b) une liste comprenant les préférences en matière de produits alimentaires; et
c) une diversité alimentaire quotidienne privilégiée, cette diversité étant choisie parmi une diversité élevée (aucun repas ne se répétant plus d'une fois sur une période de 4 jours), une diversité moyenne (aucun repas ne se répétant plus d'une fois sur une période de 3 jours) et une faible diversité (aucun repas ne se répétant plus d'une fois sur une période de 2 jours); et
d) un type de repas, ce type de repas étant choisi parmi le petit-déjeuner, le déjeuner, le dîner et la collation, et

e) les préférences de temps de préparation de repas, les préférences de temps de préparation de repas étant choisies parmi le repas qui peut être préparé entre 10 minutes et le repas qui peut être préparé entre 10 à 60 minutes, et

f) le nombre de fois par semaine où le sujet humain pratique une activité sportive, et g) l'âge, et

h) la taille, et

i) le poids actuel, et

j) le sexe et

k) le poids préféré,

où la mémoire est configurée pour stocker des données permettant d'exécuter un procédé selon la revendication 1, dans lequel le processeur est connecté à la première unité de communication, à l'écran et à la mémoire et est configuré pour exécuter le procédé selon la revendication 1;

où la première unité de communication est configurée pour communiquer des données collectées par l'appareil de l'utilisateur via Internet à une deuxième unité de communication;

un dispositif de surveillance d'activité utilisé par le sujet humain et configuré pour communiquer avec l'appareil de l'utilisateur;

la seconde unité de communication est configurée pour communiquer via Internet avec la première unité de communication du dispositif utilisateur, communiquer avec une unité de collecte de données et une unité de mise à l'échelle, et est configurée pour transmettre les données collectées par l'unité de collecte de données et l'unité de mise à l'échelle via Internet à la première unité de communication, configurée pour donner un signal via Internet à la première unité de communication concernant la nécessité de préparer et d'effectuer le petit-déjeuner, le déjeuner, le dîner ou une collation, et ajuster le signal en fonction de la préférence de repas sélectionnée pour le petit-déjeuner, le déjeuner, le dîner ou une collation, configurée pour envoyer une proposition de modification du plan de repas communiquée par l'unité de collecte de données, et via Internet à la première unité de communication, l'unité de collecte de données est configurée pour communiquer avec la seconde unité de communication, une unité de surveillance, l'unité de mise à l'échelle, une unité de suggestion, une unité de calcul des kcal quotidiennes et une unité de modification, et configurée pour envoyer la proposition de modification du plan de repas communiquée par l'unité de suggestion à la seconde unité de communication, une unité de stockage est configurée pour stocker des recettes marquées avec des produits, dans laquelle l'unité de stockage est configurée pour communiquer avec une unité de marquage;

L'unité de marquage est configurée pour communiquer avec l'unité de stockage et l'unité de mise à l'échelle; elle marque les recettes selon une ou plusieurs catégories d'objectifs sélectionnées parmi le maintien du poids, la perte de poids et la prise de poids, ainsi que les préférences de temps de préparation des repas sélectionnées parmi un repas qui peut être préparé en moins de 10 minutes et un repas qui peut être préparé entre 10 et 60 minutes, et les préférences de plan de repas sélectionnées parmi le plan végétalien, le plan végétarien, le plan composé de tous les aliments, le plan composé de tous les aliments sauf la viande et le régime paléo; elle marque également les produits en indiquant leurs valeurs nutritionnelles, notamment la quantité de glucides, de glucides-fibres, de fibres, de protéines et de lipides qu'ils contiennent pour 100 g de produit non cuit, et en précisant que le produit est une source de glucides, de glucides-fibres, de fibres, de protéines ou de lipides, selon sa valeur nutritionnelle prédominante, et définit la quantité de kcal pour chacun des quatre repas d'une journée, la quantité de kcal pour le petit-déjeuner étant de 35 %, pour le déjeuner de 35 %, pour le dîner de 20 % et pour la collation de 10 %, sur la base de la quantité totale de kcal quotidienne;

l'unité de calcul des kcal journalières est configurée pour communiquer avec l'unité de collecte de données et l'unité de mise à l'échelle et calculer une quantité de kcal journalière en fonction de l'objectif communiqué par l'unité de collecte de données;

l'unité de mise à l'échelle est configurée pour communiquer avec l'unité de marquage, l'unité de collecte de données, l'unité de calcul des kcal journalières, l'unité de modification et la seconde unité de communication.

L'unité de mise à l'échelle est configurée pour calculer la quantité totale de kcal de la recette marquée fournie par l'unité de marquage, en fonction de la quantité d'ingrédients et de leur valeur calorique, puis pour calculer la différence entre la recette marquée et la quantité de kcal nécessaire en fonction de la quantité de kcal journalière communiquée par l'unité de calcul des kcal journalières, et pour identifier les ingrédients de la recette et les classer dans l'une des catégories suivantes: glucides, glucides-fibres, fibres, protéines et lipides; et pour communiquer le plan de repas à la seconde unité de communication;

l'unité de surveillance est configurée pour communiquer avec l'unité de collecte de données et l'unité de suggestion, dans laquelle l'unité de surveillance est configurée pour surveiller les données communiquées par l'unité de collecte de données et analyser si le plan de repas établi est efficace ou inefficace et communiquer le résultat à l'unité de suggestion;

l'unité de suggestion est configurée pour communiquer avec l'unité de surveillance et l'unité de collecte de données, dans laquelle l'unité de suggestion est configurée pour envoyer une proposition de modification du plan de repas à l'unité de collecte de données dans le cas où elle aurait reçu des données de l'unité de surveillance indiquant que le plan de repas est inefficace, dans lequel, après l'acceptation de la proposition par le sujet humain concerné, l'acceptation est envoyée par Internet à la deuxième unité de communication;

l'unité de changement est configurée pour communiquer avec l'unité de collecte de données et l'unité de mise à l'échelle et est configurée pour modifier le plan de repas et communiquer un nouveau plan de repas à mettre à l'échelle à l'unité de mise à l'échelle, si l'unité de changement a communiqué l'acceptation de la proposition de modification du plan de repas à l'unité de changement;

l'unité de mise à l'échelle est configurée pour recevoir le nouveau plan de repas à mettre à l'échelle et mettre à l'échelle les recettes reçues de l'unité de marquage pour correspondre à la quantité quotidienne de kcal obtenue dans l'unité de calcul quotidienne de kcal pour chaque repas et obtenir le nouveau plan de repas, et communiquer le nouveau plan de repas à la deuxième unité de communication.

FIG.1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 110379487
- CN 109545327
- JP 2016538617 B
- US 20090144081 A
- US 7361143 B
- US 20200074884 A
- US 2014287384 A1
- CN 111584039 A